# EUROPEAN PATENT SPECIFICATION

(11) **EP 2 209 901 B1**
(45) Date of publication and mention of the grant of the patent: **17.02.2016**
(21) Application number: 08838166.0
(22) Date of filing: 09.10.2008
(51) Int. Cl.: C12P 7/10

(54) **METHODS AND COMPOSITIONS FOR ENHANCED PRODUCTION OF ORGANIC SUSTANCES FROM FERMENTING MICROORGANISMS**
VERFAHREN UND ZUSAMMENSETZUNGEN ZUR VERBESSERTEN PRODUKTION ORGANISCHER STOFFE AUS FERMENTIERENDEN MIKROORGANISMEN
PROCÉDÉS ET COMPOSITIONS POUR LA PRODUCTION OPTIMISÉE DE SUBSTANCES ORGANIQUES À PARTIR DE MICRO-ORGANISMES EN FERMENTATION

(30) Priority: 12.10.2007 US 979720 P
(43) Date of publication of application: 28.07.2010
(73) Proprietor: Danisco US Inc., Palo Alto, California 94304 (US)
(72) Inventor: LI, Mian, Palo Alto, California 94304 (US); MITCHINSON, Colin, Palo Alto, California 94304 (US); STEELE, Landon, Palo Alto, California 94304 (US)
(74) Representative: Kremer, Simon Mark
(86) International application number: PCT/US2008/079377
(87) International publication number: WO 2009/049067

(56) References cited:
- WO-A-2004/035070
- WO-A-2006/115455
- WO-A-2007/113417
- GB-A- 1 497 958
- US-A1- 2005 233 423
- LABEILLE P ET AL: "Comparative study of wheat flour saccharification and ethanol production with two glucoamylase preparations", INDUSTRIAL CROPS AND PRODUCTS, ELSEVIER, NL, vol. 6, no. 3/04, 1 August 1997 (1997-08-01), pages 291-295, XP002119202, ISSN: 0926-6690, DOI: DOI:10.1016/S0926-6690(97)00020-4
- ALFENORE S ET AL: "Improving ethanol production and viability of Saccharomyces cerevisiae by a vitamin feeding strategy during fed-batch process.", APPLIED MICROBIOLOGY AND BIOTECHNOLOGY, vol. 60, no. 1-2, October 2002 (2002-10), pages 67-72, ISSN: 0175-7598
- K.Jacques, T.P. Lyons, D.R. Kelsall: In: "The Alcohol Textbook", 31 December 1999 (1999-12-31), Nottingham University Press, Nottingham pages 73-74,

## Description

### II. INTRODUCTION

### A. Field

The present disclosure relates to production of organic substances from fermenting microorganisms using saccharification and fermentation methods and compositions.

### B. Background

As the limits of non-renewable energy resources approach, the potential of complex polysaccharides such as cellulose as a renewable energy resource is enormous. Cellulose can be converted into sugars, such as glucose, and used as an energy source by numerous microorganisms including bacteria, yeast and other fungi for industrial purposes. Utilization of cellulosic materials as a renewable carbon source depends on the development of economically feasible methods for both hydrolyzing cellulose to sugar, as well as converting those sugars to usable fuels, such as ethanol.

Cellulose can be broken down to products such as glucose, cellobiose, and other cellooligosaccharides, by enzymes called cellulases. Cellulase enzymes work synergistically to hydrolyze cellulose to glucose. Exo-cellobiohydrolases (CBHs) such as CBHI and CBHII, generally act on the ends of cellulose to generate cellobiose, while the endoglucanases (EGs) act at random locations on the cellulose. Together these enzymes hydrolyze cellulose into smaller cello-oligosaccharides such as cellobiose. Cellobiose is hydrolyzed to glucose by beta-glucosidase. Although many microorganisms are capable of degrading cellulose, only a few of these microorganisms produce significant quantities of enzymes capable of completely hydrolyzing crystalline cellulose. To date, none of these strains is also capable of efficiently converting the resulting products to industrial scale organic substances such as ethanol. This second step is typically performed using a variety of fermenting microorganism such as commercially available yeast strains to produce ethanol.

GB1497958A describes a method for the manufacture of ethanol from a cellulosic material, comprising simultaneously reacting under anaerobic conditions a cellulosic material, a cellulase, and an alcohol-producing micro-organism to obtain said ethanol, in which: the cellulase source is the entire aqueous culture broth obtained by cultivating a cellulolytic micro-organism in an aqueous nutrient medium in the presence of a cellulosic material, whereby greater yields of ethanol are obtained.

US2005/233423A1 describes methods for converting plant cell wall polysaccharides into one or more products, comprising: treating the plant cell wall polysaccharides with an effective amount of a spent whole fermentation broth of a recombinant microorganism, wherein the recombinant microorganism expresses one or more heterologous genes encoding enzymes which degrade or convert the plant cell wall polysaccharides into the one or more products.

WO2007/113417A describes nutritional supplement for alcoholic fermentation medium based on carbohydrate starting materials, in particular starchy starting materials, comprising, and preferably consisting of, an active ingredient derived from a fermentation with a mould.

WO2004/035070 describes a composition useful for inducing expression of genes whose expression is under control of an inducible promoter sequence and methods for the compositions preparation and use.

Labeille P et al., INDUSTRIAL CROPS AND PRODUCTS, vol. 6, no. 3/04, 1 August 1997, pages 291-295 describes a glucoamylase preparation from *Aspergillus niger* and compares it to a commercial preparation. Saccharified mashes obtained by both amylolytic solutions were used for ethanol production. The *Aspergillus niger* glucoamylase preparation gave similar results to the commercial one in wheat flour saccharification. Moreover, the secondary enzymatic activities associated with the *Aspergillus niger* cocktail improved bioethanol production.

While it may be ecologically desirable to develop renewable organic substances such as cellulosic ethanol, this multi-step process cannot yet compete economically with non-renewable carbon sources such as oil and natural gas. Thus, there remains a strong need to develop more efficient systems for generating organic substances from fermenting microorganisms, such as ethanol from source materials. It is therefore desired to improve the efficiency and economics of the enzymatic hydrolysis (saccharification) and fermentation of cellulosic materials.

### III. SUMMARY

The present disclosure relates methods and compositions for producing an organic substance using simultaneous saccharification and fermentation. One aspect provides producing an organic substance by simultaneous saccharification and fermentation comprising: combining, in the absence of a supplemental nitrogen source, a cellulosic substrate, a whole fermentation broth and a fermenting microorganism, and incubating the cellulosic substrate, whole fermentation broth and fermenting microorganism under conditions conducive both to hydrolysis of cellulose to glucose and/or xylose and to conversion of glucose and/or xylose to the organic substance. One aspect provides methods of producing ethanol by simultaneous saccharification and fermentation, comprising: combining, in the absence of a supplemental nitrogen source, a cellulosic substrate, a whole fermentation broth, and incubating the cellulosic substrate, whole fermentation broth and ethanologenic microorganism under conditions conducive both to hydrolysis of cellulose to glucose and to conversion of glucose to ethanol.

The present disclosure also relates to reactive compositions for the production of an organic substance from a fermenting microorganism comprising a mixture of a cellulosic substrate, whole fermentation broth and fermenting microorganism, wherein the reactive composition is substantially free of supplemental nitrogen source. Another aspect provides reactive compositions for the production of ethanol, comprising a mixture of cellulosic substrate, a whole fermentation broth, an ethanologenic microorganism, wherein the reactive composition is substantially free of a supplemental nitrogen source.

### IV. BRIEF DESCRIPTION OF THE DRAWINGS

The skilled artisan will understand that the drawings are for illustration purposes only and are not intended to limit the scope of the present teachings in any way.
FIG. 1 illustrates the resulting ethanol (ETOH) concentration (g/L) of simultaneous saccharification and fermentation of acid-pretreated bagasse in the absence of supplemental nutrients.
FIG. 2 illustrates that clarified fermentation broth must be supplemented with additional nutrients in order to achieve ethanol concentrations comparable to those from simultaneous saccharification and fermentation of acid-pretreated bagasse with a whole fermentation broth.
FIG. 3 provides a comparison of simultaneous saccharification and fermentation with a whole fermentation broth with and without supplemental nutrients.
FIG. 4 provides a comparison of saccharification of acid-pretreated bagasse using a whole fermentation broth and clarified fermentation broth supplemented with beta-glucosidase at the same amount of cellulolytic activity.

### V. DETAILED DESCRIPTION

It is to be understood that both the foregoing general description and the following detailed description are exemplary and explanatory only and are not restrictive of the compositions and methods described herein. Unless defined otherwise herein, all technical and scientific terms used herein have the same meaning as commonly understood by one of ordinary skill in the art to which this invention belongs. In this application, the use of the singular includes the plural unless specifically stated otherwise. The use of "or" means "and/or" unless state otherwise. Likewise, the terms "comprise," "comprising," "comprises," "include," "including" and "includes" are not intended to be limiting. The headings provided herein are not limitations of the various aspects or embodiments of the invention which can be had by reference to the specification as a whole. Accordingly, the terms herein are more fully defined by reference to the specification as a whole.

The present disclosure relates methods and compositions for producing an organic substance using simultaneous saccharification and fermentation.

The present disclosure also relates to methods of producing organic substances by simultaneous saccharification and fermentation comprising: (a) combining, in the absence of a supplemental nitrogen source, a cellulosic substrate, a whole fermentation broth and an fermenting microorganism; and (b) incubating the cellulosic substrate, whole fermentation broth and fermenting microorganism under conditions conducive both to hydrolysis of cellulose to glucose and/or xylose and to conversion of glucose and/or xylose to an organic substance.

Also provided herein are reactive compositions for the production of organic substances from fermenting microorganisms. In some embodiments, the reactive composition for production of organic substance from a fermenting microorganism consists essentially of a mixture of a cellulosic substrate, a whole fermentation broth, a fermenting microorganism, and water. In some embodiments, the reactive composition for production of organic substance comprises a mixture of a cellulosic substrate, whole fermentation broth and fermenting microorganism, wherein the reactive composition is substantially free of supplemental nitrogen source.

As used here, the term "cellulosic substrate" refers to any plant biomass materials containing cellulose and/or hemi-cellulose. A cellulosic substrate may also be a lignocellulosic material, which is composed of cellulose, hemi-cellulose and beta-glucans that are cross-linked with each other and with lignin. Such cellulosic substrates may also contain other materials such as pectins, proteins starch and lipids, but preferably will have cellulose, hemi-cellulose and beta-glucans as primary components.

Suitable non-limiting examples of cellulosic substrates include, but are not limited to, biomass, herbaceous material, agricultural residues, forestry residues, municipal solid waste, waste paper, and pulp and paper residues. Common forms of cellulosic substrate for use in the present invention include, but are not limited to trees, shrubs and grasses, wheat, wheat straw, sugar cane bagasse, corn, corn husks, corn kernel including fiber from kernels, products and by-products from milling of grains such as corn (including wet milling and dry milling) as well as municipal solid waste, waste paper and yard waste. The cellulosic substrate may be obtained from "virgin biomass" (such as trees, bushes, grasses, fruits, flowers, herbaceous crops, hard and soft woods.), "non-virgin biomass" (such as agricultural byproducts, commercial organic waste, construction and demolition debris, municipal solid waste and yard waste), or "blended biomass," which is a mixture of virgin and non-virgin biomass

In some embodiments, the cellulosic substrate includes wood, wood pulp, papermaking sludge, paper pulp waste streams, particle board, corn stover, corn fiber, rice, paper and pulp processing waste, woody or herbaceous plants, fruit pulp, vegetable pulp, pumice, distillers grain, grasses, rice hulls, sugar cane bagasse, cotton, jute, hemp, flax, bamboo, sisal, abaca, straw, corn cobs, distillers grains, leaves, wheat straw, coconut hair, algae, switchgrass, and mixtures thereof.

The cellulosic substrate can be used as is or may be subjected to pretreatment using conventional methods known in the art. Such pretreatments include chemical, physical, and biological pretreatment. For example, physical pretreatment techniques can include without limitation various types of milling, crushing, steaming/steam explosion, irradiation and hydrothermolysis. Chemical pretreatment techniques can include without limitation dilute acid, alkaline, organic solvent, ammonia, sulfur dioxide, carbon dioxide, and pH-controlled hydrothermolysis. Biological pretreatment techniques can include without limitation applying lignin-solubilizing microorganisms.

In the present disclosure, the whole fermentation broth can be prepared from any filamentous fungi that is useful for the degradation of a cellulosic substrate. The term "filamentous fungi" means any and all filamentous fungi recognized by those of skill in the art, and includes naturally occurring filamentous fungi, filamentous fungi with naturally occurring or induced mutations, and filamentous fungi that have been genetically modified. In general, filamentous fungi are eukaryotic microorganisms and include all filamentous forms of the subdivision Eumycotina and Oomycota. These fungi are characterized by a vegetative mycelium with a cell wall composed of chitin, beta-glucan, and other complex polysaccharides. In some embodiments, the filamentous fungi of the present teachings are morphologically, physiologically, and genetically distinct from yeasts.

In some embodiments, the whole fermentation broth is prepared from a *Acremonium, Aspergillus, Emericella, Fusarium, Humicola, Mucor, Myceliophthora, Neurospora, Scytalidium, Thielavia, Tolypocladium,* or *Trichoderma* species or species derived therefrom.

In some embodiments, the whole fermentation broth is prepared from fermentation of *Aspergillus aculeatus, Aspergillus awamori, Aspergillus foetidus, Aspergillus japonicus, Aspergillus nidulans, Aspergillus niger, or Aspergillus oryzae. In another aspect, whole broth is prepared from Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides,* or *Fusarium venenatum.* In another aspect, the whole fermentation broth is prepared from fermentation of *Humicola insolens, Humicola lanuginosa, Mucor miehei, Myceliophthora thermophila, Neurospora crassa, Scytalidium thermophilum,* or *Thielavia terrestris.* In another aspect, the whole fermentation broth is prepared from fermentation *Trichoderma harzianum, Trichoderma koningii, Trichoderma longibrachiatum, Trichoderma reesei e.g.,* RL-P37 (Sheir-Neiss et al., Appl. Microbiol. Biotechnology, 20 (1984) pp. 46-53; Montenecourt B.S., Can., 1-20, 1987), QM9414 (ATCC No. 26921), NRRL 15709, ATCC 13631, 56764, 56466, 56767, or *Trichoderma viride* e.g., ATCC 32098 and 32086.

In some embodiments, the whole fermentation broth is prepared from fermentation of filamentous fungi including, but not limited to the following genera: *Aspergillus, Acremonium, Aureobasidium, Beauveria, Cephalosporium, Ceriporiopsis, Chaetomium paecilomyces, Chrysosporium, Claviceps, Cochiobolus, Cryptococcus, Cyathus, Endothia, Endothia mucor, Fusarium, Gilocladium, Humicola, Magnaporthe, Myceliophthora, Myrothecium, Mucor, Neurospora, Phanerochaete, Podospora, Paecilomyces, Pyricularia, Rhizomucor, Rhizopus, Schizophylum, Stagonospora, Talaromyces, Trichoderma, Thermomyces, Thermoascus, Thielavia, Tolypocladium, Trichophyton,* and *Trametes pleurotus.* In some embodiments, the whole fermentation broth is prepared from fermentation of filamentous fungi including, but are not limited to the following: *A. nidulans, A. niger, A. awomari, A. aculeatus, A. kawachi* e.g., NRRL 3112, ATCC 22342 (NRRL 3112), ATCC 44733, ATCC 14331 and strain UVK 143f, *A. oryzae,* e.g., ATCC 11490, *N. crassa, Trichoderma reesei,* e.g., NRRL 15709, ATCC 13631, 56764, 56765, 56466, 56767, and *Trichoderma viride,* e.g., ATCC 32098 and 32086. In a preferred implementation, the whole fermentation broth is prepared from fermentation of a *Trichoderma* species. A particularly preferred species and strain for use in the present invention is Trichoderma reesei RutC30 whole cellulase, which is available from the American Type Culture Collection as Trichoderma reesei ATCC 56765.

As described above the whole fermentation broth can be prepared from fermentation of non-recombinant and/or recombinant filamentous fungi. In some embodiments the filamentous fungus is a recombinant filamentous fungus comprising one or more genes which can be homologous or heterologous to the filamentous fungus. In some embodiments, the filamentous fungus is a recombinant filamentous fungus comprising one or more genes which can be homologous or heterologous to the filamentous fungus wherein the one or more genes encode enzymes that can degrade a cellulosic substrate. Genes encoding cellulosic material degrading enzymes are know to those skilled in the art. Suitable non-limiting examples of genes that encode enzymes that degrade cellulosic substrates include endoglucanases, cellobiohydrolases, glucohydrolases, beta-glucosidases, xyloglucanases, xylanases, xylosidases, alpha-arabinofuranosidases, alpha-glucuronidases, acetyl xylan esterases, mannanases, mannosidases, alpha-galactosidases, mannan acetyl esterases, galactanases, arabinanases, pectate lyases, pectin lyases, pectate lyases, polygalacturonases, pectin acetyl esterases, pectin methyl esterases, alpha-arabinofuranosidases, beta-galactosidases, galactanases, arabinanases, alpha-arabinofuranosidases, rhamnogalacturonases, rhamnogalacturonan lyases, and rhamnogalacturonan acetyl esterases, xylogalacturonosidases, xylogalacturonases, rhamnogalacturonan lyases, lignin peroxidases, manganese-dependent peroxidases, and laccases.

In some embodiments, the whole fermentation broth may be supplemented with one or more enzyme activities that are not expressed endogenously, or expressed at relatively low level by the filamentous fungi, to improve the degradation of the cellulosic substrate, for example, to fermentable sugars such as glucose or xylose. The supplemental enzyme(s) can be added as a supplement to the whole fermentation broth and the enzymes may be a component of a separate whole fermentation broth, or may be purified, or minimally recovered and/or purified. Suitable, non-limiting examples of supplemental enzymes include cellobiohydrolases, endoglucanase, beta-glucosidase, endo-beta-1,3(4)-glucanase, glucohydrolase, xyloglucanase, xylanase, xylosidase, arabinofuranosidase, alpha-glucuronidase, acetyl xylan esterase, mannanase, mannosidase, alpha-galactosidase, mannan acetyl esterase, galactanase, arabinanase, pectate lyase, pectinase lyase, pectate lyase, polygalacturonase, pectin acetyl esterase, pectin methyl esterase, beta-galactosidase, galactanase, arabinanase, alpha-arabinofuranosidase, rhamnogalacturonase, ferrulic acid esterases rhamnogalacturonan lyase, rhamnogalacturonan acetyl esterase, xylogalacturonosidase, xylogalacturonase, rhamnogalacturonan lyase, lignin peroxidases, manganese-dependent peroxidases, hybrid peroxidases, with combined properties of lignin peroxidases and manganese-dependent peroxidases, glucoamylase, amylase, protease, and laccase.

In some embodiments, the whole fermentation broth comprises a whole fermentation broth of a fermentation of a recombinant filamentous fungi over-expressing an enzyme(s) to improve the degradation of the cellulosic substrate. Alternatively, the whole fermentation broth can comprise a mixture of a whole fermentation broth of a fermentation of a non-recombinant filamentous fungi and a recombinant filamentous fungi over-expressing an enzyme(s) to improve the degradation of the cellulosic substrate.

In some embodiments, the whole fermentation broth comprises a whole fermentation broth of a fermentation of a filamentous fungi over-expressing β-glucosidase. Alternatively, the whole fermentation broth for use in the present methods and reactive compositions can comprise a mixture of a whole fermentation broth of a fermentation of a non-recombinant filamentous fungi and a whole fermentation broth of a fermentation of a recombinant filamentous fungi over-expressing a β-glucosidase.

The term "beta-glucosidase" is defined herein as a beta-D-glucoside glucohydrolase classified as EC 3.2.1.21, and/or those in certain GH families, including, but not limited to, those in GH families 1, 3, 7, 9 or 48, which catalyzes the hydrolysis of cellobiose with the release of beta-D-glucose. The over-expressed beta-glucosidase can be from the same or different species than that of the host filamentous fungi. Notably, the over-expressed beta-glucosidase need not be a fungal beta-glucosidase.

In some embodiments, the beta-glucosidase can be produced by expressing a gene encoding beta-glucosidase. For example, beta-glucosidase can be secreted into the extracellular space e.g., by Gram-positive organisms, (such as *Bacillus* and *Actinomycetes*), or eukaryotic hosts (e.g., *Trichoderma, Aspergillus, Saccharomyces,* and *Pichia*). It is to be understood, that in some embodiments, that beta-glucosidase can be over-expressed in a recombinant microorganism relative to the native levels. In some embodiments, if a host cell is employed for expression of the beta-glucosidase, the cell may be genetically modified to reduce expression of one or more proteins that are endogenous to the cell. In one embodiment, the cell may contain one or more native genes, particularly genes that encode secreted proteins that have been deleted or inactivated. For example, one or more protease-encoding genes (e.g. an aspartyl protease-encoding gene; see Berka et al, Gene 1990 86:153-162 and USP 6,509,171) or cellulase-encoding genes may be deleted or inactivated. In one embodiment, the Trichoderma sp. host cell may be a T. reesei host cell contain inactivating deletions in the cbh1, cbh2 and egl1, and egl2 genes, as described in WO 05/001036. The nucleic acids encoding beta-glucosidase may be present in the nuclear genome of the Trichoderma sp. host cell or may be present in a plasmid that replicates in the Trichoderma host cell, for example.

Preferred examples of beta-glucosidase that can be used include beta-glucosidase from Aspergillus aculeatus (Kawaguchi et al., 1996, Gene 173: 287-288), Aspergillus kawachi (Iwashita et al., 1999, Appl. Environ. Microbiol. 65: 5546-5553), Aspergillus oryzae (WO 2002/095014), Cellulomonas biazotea (Wong et al., 1998, Gene 207: 79-86), Saccharomycopsis fibuligera (Machida et al., 1988, Appl. Environ. Microbiol. 54: 3147-3155), Schizosaccharomyces pombe (Wood et al., 2002, Nature 415: 871-880), and Trichoderma reesei beta-glucosidase 1 (US Patent No. 6,022,725), Trichoderma reesei beta-glucosidase 3 (US Patent No.6,982,159), Trichoderma reesei beta-glucosidase 4 (US Patent No. 7,045,332), Trichoderma reesei beta-glucosidase 5 (US Patent No. 7,005,289), Trichoderma reesei beta-glucosidase 6 (USPN 20060258554) Trichoderma reesei beta-glucosidase 7 (USPN 20040102619).

In a preferred implementation, the whole fermentation broth has at least 400 pNPG U/g β-glucosidase activity, wherein one pNPG unit of activity denotes 1 µmol of nitrophenol liberated from para-nitrophenyl-B-D-glucopyranoside in 10 minutes at 50°C and pH 4.8. The activity of the beta-glucosidase and the activity of the whole cellulase preparation can be determined using methods known in the art. In this context, the following conditions can be used. Beta-glucosidase activity can determined by any means know in the art, such as the assay described by Chen, H.; Hayn, M.; Esterbauer, H. "Purification and characterization of two extracellular β-glucosidases from Trichoderma reesei", Biochimica et Biophysica Acta, 1992, 1121, 54-60. One pNPG denotes 1 mol of Nitrophenol liberated from para-nitrophenyl-B-D-glucopyranoside in 10 minutes at 50 ºC (122ºF) and pH 4.8. Cellulase activity of the whole cellulase preparation may be determined using carboxymethyl cellulose (CMC) as a substrate. Determination of whole cellulase activity, measured in terms of CMC activity. This method measures the production of reducing ends created by the enzyme mixture acting on CMC wherein 1 unit is the amount of enzyme that liberates 1 mol of product/minute (Ghose, T. K., Measurement of Cellulse Activities, Pure & Appl. Chem. 59, pp. 257-268, 1987).

In some implementations, the whole fermentation broth has at least 2500 CMC U/g endoglucancase activity, wherein one CMC unit of activity liberates 1 µmol of reducing sugars in one minute at 50°C and pH 4.8. While the overall cellulase activity is important to the present invention, the ratio of overall cellulase activity to beta-glucosidase activity can be important, as the beta-glucosidase hydrolyzes an end product that otherwise negatively affects the activity of other cellulases. In some implementations, the whole broth comprises an enzyme activity ratio in a range from about 0.5 to 25 pNPG/CMC units. In some embodiments, enzyme activity ratio is from about 1 to 20 pNPG/CMC units, or from about 1.5 to 15 pNPG/CMC units, or from about 2 to 10 pNPG/CMC units, or from about 2.5 to 8 pNPG/CMC units, from about 3 to 7 pNPG/CMC units, or from about 3.5 to 6.5 pNPG/CMC units, or from about 4 to 6 pNPG/CMC unit, or from about 4.5 to 5.5 pNPG/CMC units, or from about 5 to 6 pNPG/CMC. Especially suitable are, for example, ratios of about 5.5 pNPG/CMC units.

The appropriate dosage levels and operating conditions will be apparent to those of skill in the art, especially in light of the detailed disclosure provided herein. Optimum dosage levels of the whole fermentation broth will vary considerably depending upon the cellulosic substrate and the pretreatment technologies used. Operating conditions such as pH, temperature and reaction time may also affect rates of ethanol production. Preferably, the reactive composition contains 0.006 to 6 mL of whole fermentation broth per gram of cellulose, more preferably 0.015 to 1.5 mL of whole fermentation broth per gram of cellulose and most preferably 0.03 to 0.6 mL whole fermentation broth per gram of cellulose. Alternatively, the amount of whole fermentation broth can be determined based on the total amount of biomass substrate in the system. In such a case, the reactive composition preferably contains 0.003 to 3 mL whole fermentation broth per gram of biomass substrate, more preferably, 0.075 to 0.75 mL whole fermentation broth per gram of biomass substrate and more preferably 0.015 to 0.3 mL whole fermentation broth per gram of biomass substrate. In another implementation, the whole fermentation broth can be added in amounts effective from about 0.3 to 300.0% wt. of biomass substrate solids, more preferably from about 0.75% to 75% wt. of biomass substrate solids, and most preferably from about 1.5% to 30% wt. of biomass substrate solids. Alternatively, the amount of whole fermentation broth can be determined based on the total amount of whole fermentation broth derived cell mass provided to the system. In such a case, the reactive composition preferably contains 0.0001 to 0.1 gm of whole fermentation broth derived cell mass per gram of biomass substrate, more preferably, 0.00025 to 0.025 gm of whole fermentation broth derived cell mass per gram of biomass substrate, and more preferably, 0.0005 to 0.01 gm of whole fermentation broth derived cell mass per gram of biomass substrate.

As described herein, the whole fermentation broth can be from any filamentous fungi cultivation method known in the art resulting in the expression of enzymes capable of hydrolyzing a cellulosic substrate. Fermentation can include shake flask cultivation, small- or large-scale fermentation, such as continuous, batch, fed-batch, or solid state fermentations in laboratory or industrial fermenters performed in a suitable medium and under conditions allowing cellulase to be expressed. Typically, the whole fermentation broth includes cellulolytic enzymes including, but are not limited to: (i) endoglucanases (EG) or 1,4--d-glucan-4-glucanohydrolases (EC 3.2.1.4), (ii) exoglucanases, including 1,4--d-glucan glucanohydrolases (also known as cellodextrinases) (EC 3.2.1.74) and 1,4--d-glucan cellobiohydrolases (exo-cellobiohydrolases, CBH) (EC 3.2.1.91), and (iii) beta-glucosidase (BG) or beta-glucoside glucohydrolases (EC 3.2.1.21).

Generally, the filamentous fungi is cultivated in a cell culture medium suitable for production of enzymes capable of hydrolyzing a cellulosic substrate. The cultivation takes place in a suitable nutrient medium comprising carbon and nitrogen sources and inorganic salts, using procedures known in the art. Suitable culture media, temperature ranges and other conditions suitable for growth and cellulase production are known in the art.

Preferably, the fermentation of the filamentous fungi is conducted in such a manner that the carbon-containing substrate can be controlled as a limiting factor, thereby providing good conversion of the carbon-containing substrate to cells and avoiding contamination of the cells with a substantial amount of unconverted substrate. The latter is not a problem with water-soluble substrates, since any remaining traces are readily washed off. It may be a problem, however, in the case of non-water-soluble substrates, and require added product-treatment steps such as suitable washing steps.

The whole fermentation broth can be prepared by growing the filamentous fungi to stationary phase and maintaining the filamentous fungi under limiting carbon conditions for a period of time sufficient to express the one or more cellulases or beta-glucosidases. Once enzymes, such as cellulases, are secreted by the filamentous fungi into the fermentation medium, the whole fermentation broth can be used.

The whole fermentation broth comprises filamentous fungi. In some embodiments, the whole fermentation broth comprises the unfractionated contents of the fermentation materials derived at the end of the fermentation. Typically the whole fermentation broth comprises the spent culture medium and cell debris present after the filamentous fungi is grown to saturation, incubated under carbon-limiting conditions to allow protein synthesis (particularly expression of cellulases and/or glucosidases). In some embodiments, the whole fermentation broth comprises the spent cell culture medium, extracellular enzymes and filamentous fungi. In some embodiments, the filamentous fungi present in whole fermentation broth can be lysed, permeabilized, or killed using methods known in the art to produce a cell-killed whole fermentation broth.

In some implementations, the whole fermentation broth is a cell-killed whole fermentation broth wherein the whole fermentation broth containing the filamentous fungi cells are lysed or killed. In some embodiments, the cells are killed by lysing the filamentous fungi by chemical and or/pH treatment to generate the cell-killed whole broth of a fermentation of the filamentous fungi. In some embodiments, the cells are killed by lysing the filamentous fungi by chemical and or/pH treatment and adjusting the pH of the cell-killed fermentation mix to a pH between about 4 and 6, inclusive, to generate the cell-killed whole broth of a fermentation of the filamentous fungi.

Additional preservatives and or bacteriostatic agents optionally can be added to the whole fermentation broth or the cell-killed whole fermentation broth, including, but no limited to, sorbitol, sodium chloride, potassium sorbate, and others known in the art.

While not being bound to a theory, it is believed that the unclarified, whole fermentation broth provides residual nutrients to the ethanologenic microorganism. This may lead to faster ethanol fermentations and improve ethanol yields. The ability to eliminate the need to provide a nutrient broth, or reduce the amount of supplemental nutrients, to the ethanologenic microorganism in addition to the saccharified cellulose, certainly will result in decreased cost of raw materials for the ethanol fermentation process.

The methods and compositions described herein are absent or substantially free of supplemental nitrogen and/or nutrient source for the fermenting microorganism. In some embodiments, the methods and compositions are absent or substantially free of yeast extract, peptone, and/or urea. It is understood to one of ordinary skill in the art that the methods and compositions of the invention can be absent or substantially free of supplemental nitrogen source, however, trace amounts of nitrogen and/or nutrient source may be present as impurities or added in such an amount that would not substantially increase the nutrient value of the whole fermentation broth to the fermenting microorganism.

The methods and compositions described herein can reduce the amount and/or type of supplemental nitrogen source for the fermenting microorganism. In some embodiments, the methods and compositions of the present invention can reduce the amount of yeast extract, peptone, and/or urea for the fermenting microorganism.

It is understood to one of ordinary skill in the art that the methods and compositions of the invention can be used reduce the amount and/or type of supplemental nitrogen source for the fermenting microorganism.

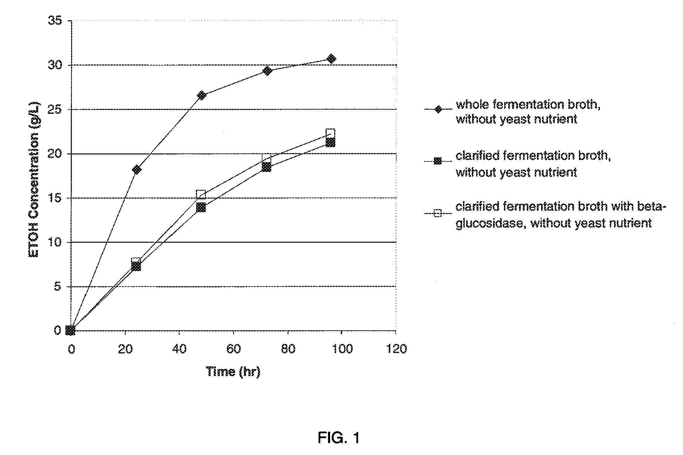

This discovery that whole fermentation broth has nutritive value to ethanologenic microorganisms is contrary to common expectations, as the nutritive value of the fermentation media is expected to be depleted or spent during fermentation of the filamentous fungi. Some studies have found that unfiltered cellulase broths demonstrate enhanced ethanol production as compared to filtered broths. However, it has been postulated that the enhanced ethanol production is due to additional beta-glucosidase attached to the cells walls of filamentous fungi that is removed during a filtration process. Schell, et al. "Whole Broth Cellulase Production for Use in Simultaneous Saccharification and Fermentation of Cellulase to Ethanol," Appl. Biochem. Biotech. 24/25:287-298 (1990). Unexpectedly, the inventors have discovered that whole fermentation broth can replace the supplemental nitrogen source (typically in the form of peptone, tryptone, yeast extract or urea) that common practice requires as a nutritional source to the ethanologenic microorganism, such as yeast. Supplementation of a clarified fermentation broth with beta-glucosidase alone cannot achieve the nutritive benefits of the whole fermentation broth (see FIG. 1 and the Examples below). Thus one aspect provides a method wherein the cellulosic substrate, whole fermentation broth and ethanologenic microorganism are combined and incubated in the absence of a supplemental nitrogen source. In other implementation, the cellulosic substrate, whole broth and ethanologenic microorganism are combined and incubated in the absence of any supplemental nutrient source.

One aspect provides methods of producing ethanol by simultaneous saccharification and fermentation, the method comprising: (a) combining, in the absence of a supplemental nitrogen source, a cellulosic substrate, a whole fermentation broth; and (b) incubating the cellulosic substrate, whole fermentation broth and ethanologenic microorganism under conditions conducive both to hydrolysis of cellulose to glucose and to conversion of glucose to ethanol.

One aspect provides methods of producing ethanol by simultaneous saccharification and fermentation comprising: (a) combining, in the absence of a supplemental nitrogen source, a cellulosic substrate, a whole fermentation broth and an ethanologenic microorganism; and (b) incubating the cellulosic substrate, whole fermentation broth and ethanologenic microorganism under conditions conducive both to hydrolysis of cellulose to glucose and/or xylose and to conversion of glucose and/or xylose to ethanol.

As used herein, fermenting microorganism means any microorganism suitable for use in a desired fermentation process for the production of organic substances. Suitable non limiting fermenting microorganisms are able to ferment or convert, sugars, such as glucose, xylose, galactose, arabinose, mannose, or oligosaccharides, into the desired fermentation product or products. Suitable non-limiting examples of fermenting microorganisms include fungal organisms, such as yeast, and bacteria. In a preferred embodiment, fermenting microorganism is an ethanologenic microorganism. The term "ethanolagenic'" is intended to include the ability of a microorganism to produce ethanol from a carbohydrate as a primary fermentation product. However, it is well known in the art that the ethanologenic organisms described herein can also be used to produce other organic substances. The term is intended to include naturally occurring ethanologenic organisms, ethanologenic organisms with naturally occurring or induced mutations, and ethanologenic organisms that have been genetically modified. While hydrolyzing the cellulosic material to glucose and other small saccharides is an important step, simultaneous saccharification and fermentation (SSF) relies upon a live culture of an ethanologenic microorganism to transform these sugars to ethanol. In some implementations, the ethanologenic microorganism is a yeast cell, such as *Saccharomyces cerevisiae, S. uvarum, Kluyveromyces fagilis, candida pseudotropicalis,* and *Pachysolen tannophilus,* that can efficiently ferment glucose to ethanol. Preferred strains include, but are not limited to, S. *cerevisiae* D_{5A} (ATCC200062), *S. cerevisiae* Y567 (ATCC24858), ACA 174 (ATCC 60868), MY91 (ATCC 201301), MY138 (ATCC 201302), C5 (ATCC 201298), ET7 (ATCC 201299), LA6 ATCC 201300), OSB21 (ATCC 201303), F23 (S. globosus ATCC 90920), ACA 174 (ATCC 60868), A54 (ATCC 90921), NRCC 202036 (ATCC 46534), ATCC 24858, ATCC 24858, G 3706 (ATCC 42594), NRRL, Y-265 (ATCC 60593), Sa28 (ATCC 26603),and ATCC 24845-ATCC 24860. Other non-cerevisiae yeast strains suitable for use in the present invention include *Pichia pastoris* (tozony ID 4922), *S*. *pastorianus* SA 23 (S. carlsbergensis ATCC 26602), *S*. *pastorianus* (S. *carlsbergensis* ATCC 2345), Candida acidothermophilum (Issatchenkia orientalis, ATCC 20381). In some embodiments, the ethanologenic microorganism is a recombinant yeast strain. Suitable recombinant yeast may contain genes encoding xylose reductase, xylitol dehydrogenase and/or xylulokinase (see for example, USPN 5,789,210).

In some implementations, the ethanologenic microorganism is a bacterial cell, preferably Gram-negative, facultatively anaerobic, and from the family *Enterobacteriaceae.* In another related embodiment, the ethanologenic microorganism is of the genus *Escherichia* or *Klebsiella* and, preferably, is the strain *E. coli* B, *E*. *coli* DH5α, *E*. *coli* KO4 (ATCC 55123), *E. coli* KO11 (ATCC 55124), *E*. *coli* KO12 (ATCC 55125), *E. coli* LY01, *K. oxytoca* M5A1, or *K*. *oxytoca* P2 (ATCC 55307). In some embodiments, the ethanologenic microorganism is a Zymomonas species, or derived from Zymomonas mobilis (ATCC31821). In some embodiments, a recombinant Zymomonas strain may contain genes encoding xylose isornerase, xylulokinase, transaldolase and transketolase, for example.

Fermenting microorganisms are typically added to the hydrolysate and the fermentation is allowed to proceed for 12-96 hours, such as 30 - 80 hours. The temperature is typically between 26-40 °C., in particular at about 32 °C, and at pH 3-6.

Following the fermentation, the organic substance of interest is recovered by any method known in the art. Such methods include, but are not limited to distillation, extraction, chromatography, electrophoretic procedures, differential solubility. For example, in an ethanol fermentation, the alcohol is separated and purified by conventional methods of distillation. The ethanol obtained according to the process of the invention may be used as fuel ethanol, drinking ethanol or as industrial ethanol.

Aspects of the present teachings may be further understood in light of the following examples, which should not be construed as limiting the scope of the present teachings. It will be apparent to those skilled in the art that many modifications, both to materials and methods, may be practiced without departing from the present teachings.

### VI. EXAMPLES

### C. Example 1: Preparation of Whole Fermentation Broth

*Preparation of glucose*/*sophorose:* 60% (w/w) glucose solution was dissolved and sterilized for 30 minutes at 121ºC. The temperature was decreased to 65ºC and 10 g of total protein (whole cellulase previously produced by *T. reesei*)/L was added. The mixture was agitated slowly and held at 65ºC for 3 days. The sophorose content was measured at 12 g/L in this 60% glucose solution.

0.8 L of media was inoculated with 1.5 ml *Trichoderma reesei* RLP-37 frozen spore suspension as a seed flask. This flask was split into two 0.4L portions and transferred to 2x7L of fermentation media in two different 14L Biolafitte fermentors after 48 hours. The growth media had the following composition:

| Media component | g/L |
|---|---|
| KH₂PO₄ | 4 |
| (NH₄)2SO₄ | 6.35 |
| MgSO₄-7H₂O | 2 |
| CaCl₂-2H₂O | 0.53 |
| Glucose/sophorose | 350 |
| Corn Steep Solids (Roquette) | 6.25 |
| Trace elements* | 1 ml/ L |

| | |
|---|---|
| Trace elements*: 5 g/L FeSO₄-7H₂O; 1.6 g/L MnSO₄-H₂O; 1.4 g/L ZnSO₄-7H₂O. | |

The fermentor was run at 25ºC, 750 RPM and 8 standard liters per minute (SLM) airflow.

The batched glucose was exhausted at approximately 20 hours at which point the cells stopped growing and a carbon limiting feed was begun. A 40% glucose/sophorose feed was added at 0.25g/minute. Total protein, which is directly correlated with cellulase production (based upon our comparison of total extracellular protein vs cellulase activity), was induced just after the batch phase. The cells were killed by lysis by a combination of chemical and pH treatment. If desirable after lysis, the killed whole fermentation broth can be brought to a more neutral pH, for example, between about pH 4 and pH 6.

### D. Example 2: Preparation of Clarified Fermentation Broth

Filamentous fungi were grown as described in Example 1, above. Rather than lysing the cells, the contents of the whole fermentation broth were filtered to remove cells and large cell debris to make a clarified fermentation broth. As the cellulases and glucosidases of interest are secreted by the *Trichoderma* cells, the cellulolytic activity is retained in the clarified fermentation broth. The enzymes contained in clarified fermentation broth were then concentrated by ultrafiltration with a 10 kDa cutoff membrane.

### E. Example 3: Simultaneous Saccharification and Fermentation

Simultaneous saccharification and fermentation (SSF) was carried out in duplicate under standard yeast fermentation conditions (e.g. Thermosacc yeast, pH 5.0, 38 °C) in a 250 ml flask. In a typical experiment, the acid-pretreated bagasse was adjusted to 7% cellulose loading using 20mM sodium citrate buffer (pH 5.0). Yeast nutrients were added to obtain the final concentrations of 1.0 g/L yeast extract, 1.0 g/L peptone, and 1.0 g/L urea. Whole fermentation broth or clarified fermentation broth (to a concentration of 0.4 CMC U/g acid-treated sugar cane bagasse) was added simultaneously with yeast to start the fermentation. When supplemented, the clarified fermentation broth has a final specific activity for β-glucosidase of 0.063 pNPG/g acid treated bagasse. The whole fermentation broth has a similar β-glucosidase activity. The flasks were agitated at 150 rpm. Samples were withdrawn at different time intervals and analyzed for ethanol, glycerol, acetic acid, lactic acid and residual sugars by HPLC method.

FIG. 1 showed that when there was no yeast nutrient, SSF with a whole fermentation broth provided a much higher concentration of ethanol than those using a clarified fermentation broth (with or without with beta-glucosidase). For example, in 96 hours, ethanol concentration was 30.7 g/L using whole fermentation broth as compared to 22.2 g/L using clarified fermentation broth supplemented with beta-glucosidase.

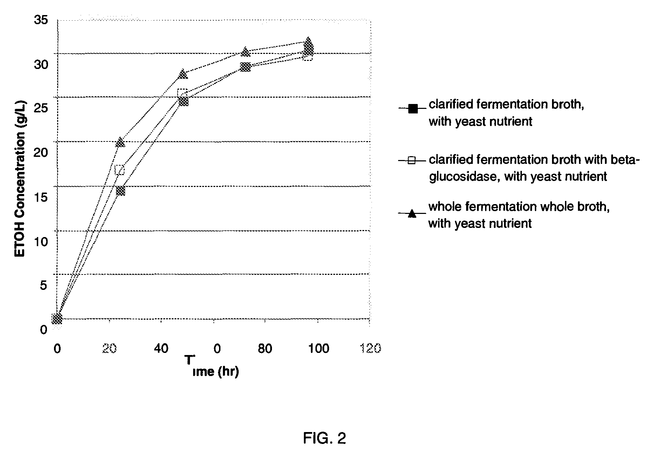

FIG. 2 compared the SSF performance using whole fermentation broth with that using clarified fermentation broth. In all cases, yeast nutrients were added at the same level. It can be seen that whole fermentation broth resulted in increased fermentation rate and slightly higher ethanol yield.

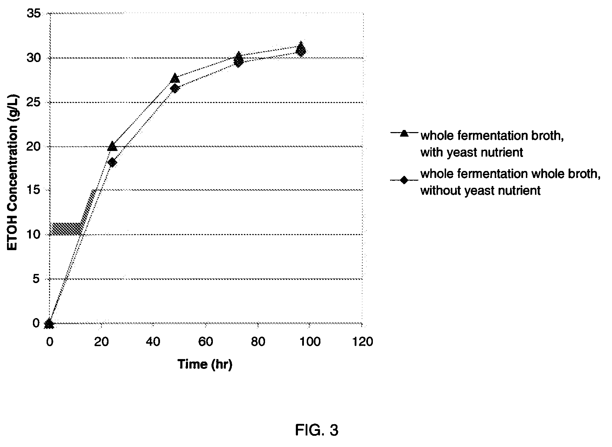

FIG. 3 showed the comparison of SSF performance using whole fermentation broth product with and without yeast nutrients. It can be seen that SSF performance was comparable with each other. In 96 hours, ethanol concentration was 31.3 g/L with yeast nutrient while the concentration was 30.7 g/L without yeast nutrient. The results suggested that yeast was able to use the nitrogen source from the whole fermentation broth for fermentation to ethanol.

### F. Example 4: Saccharification

Saccharification of acid-pretreated bagasse was carried out in duplicate in a 250 ml flask. In a typical experiment, the acid-pretreated bagasse was adjusted to 7% cellulose loading using 20mM sodium citrate buffer (pH 5.0). Whole fermentation broth cellulase or clarified fermentation broth supplemented with beta-glucosidase was added to start the enzymatic hydrolysis. The same amount of cellulolytic activity was used, namely, 0.4 CMC U/g acid-pretreated bagasse and 0.063 pNPG/g acid-pretreated bagasse, respectively. The flasks were agitated at 150 rpm. Samples were withdrawn at different time intervals and analyzed for glucose, cellobiose, and xylose by HPLC method.

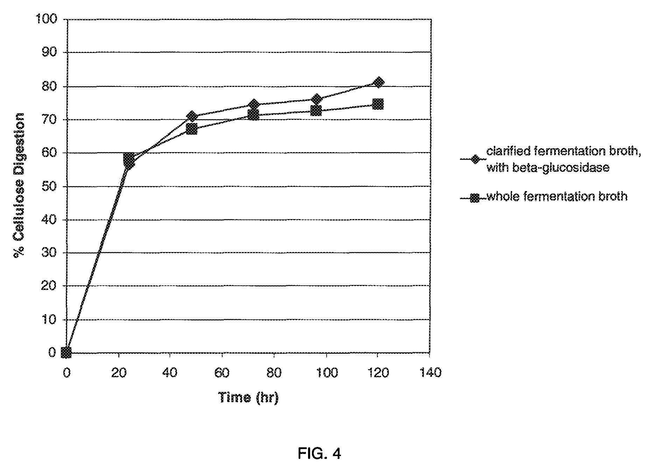

FIG. 4 illustrates that the cellulolytic capacity of the whole fermentation broth and clarified fermentation broth supplemented with beta-glucosidase is essentially identical, as evidenced by the comparable saccharification of the acid treated bagasse. Therefore, the enhanced ethanol production by whole fermentation broth is not due to beta-glucosidase, as proposed by Schell, et al. "Whole Broth Cellulase Production for Use in Simultaneous Saccharification and Fermentation of Cellulase to Ethanol," Appl. Biochem. Biotech. 24/25:287-298 (1990).

## Claims

1. A method of producing an organic substance by simultaneous saccharification and fermentation comprising:
(a) combining, in the absence of a supplemental nitrogen source, a cellulosic substrate, a whole fermentation broth and a fermenting microorganism; and
(b) incubating the cellulosic substrate, whole fermentation broth and fermenting microorganism under conditions conducive both to hydrolysis of cellulose to glucose and/or xylose and to conversion of glucose and/or xylose to organic substance,
wherein the whole fermentation broth is prepared by cultivation of a filamentous fungi useful for the degradation of a cellulosic substrate.

2. The method of Claim 1, wherein the cellulosic substrate comprises one or more cellulose sources selected from the group consisting of wood, wood pulp, papermaking sludge, paper pulp waste streams, particle board, corn stover, corn fiber, corn cob, rice, paper and pulp processing waste, woody or herbaceous plants, fruit pulp, vegetable pulp, pumice, distillers grain, grasses, rice hulls, sugar cane bagasse, cotton, jute, hemp, flax, bamboo, sisal, abaca, straw, corn cobs, leaves, wheat straw, coconut hair, algae, switchgrass, and mixtures thereof.

3. The method of Claim 1, wherein the cellulosic substrate is mechanically or chemically pretreated.

4. The method of Claim 1, wherein the fermenting microorganism is a yeast or bacterial cell.

5. The method of Claim 1, wherein the fermenting microorganism is an ethanologenic microorganism.

6. The method of Claim 1, wherein the organic substance is alcohol.

7. The method of Claim 1, wherein the organic substance is ethanol.

8. A reactive composition for production of an organic substance comprising a mixture of a cellulosic substrate, whole fermentation broth and fermenting microorganism, wherein the reactive composition is free of supplemental nitrogen source,
wherein the whole fermentation broth is prepared by cultivation of a filamentous fungi useful for the degradation of a cellulosic substrate.

9. The reactive composition of claim 8, wherein the cellulosic substrate comprises one or more cellulose source selected from the group consisting of wood, wood pulp, papermakingsludge, paper pulp waste streams, particle board, corn stover, corn fiber, corn cob, rice, paper and pulp processing waste, woody or herbaceous plants, fruit pulp, vegetable pulp, pumice, distillers grain, grasses, rice hulls, sugar cane bagasse, cotton, jute, hemp, flax, bamboo, sisal, abaca, straw, corn cobs, leaves, wheat straw, coconut hair, algae, switchgrass, and mixtures thereof.

10. The reactive composition of claim 8, wherein the cellulosic substrate is mechanically or chemically pretreated.

11. The reactive composition of claim 8, wherein the fermenting microorganism is a yeast or bacterial cell.

12. The reactive composition of claim 8, wherein the fermenting microorganism is an ethanologenic microorganism.

## Patentansprüche

1. Verfahren zum Erzeugen einer organischen Substanz durch gleichzeitige Verzuckerung und Fermentation, umfassend:
(a) Vereinigen, in Abwesenheit einer ergänzenden Stickstoffquelle, eines Cellulosesubstrats, einer Gesamtfermentationsbrühe und eines fermentierenden Mikroorganismus; und
(b) Inkubieren des Cellulosesubstrats, der Gesamtfermentationsbrühe und des fermentierenden Mikroorganismus unter Bedingungen, förderlich sowohl für Hydrolyse von Cellulose zu Glucose und/oder Xylose als auch für Umwandlung von Glucose und/oder Xylose zu organischer Substanz,
wobei die Gesamtfermentationsbrühe durch Kultivierung eines Fadenpilzes, verwendbar für den Abbau eines Cellulosesubstrats, hergestellt wird.

2. Verfahren nach Anspruch 1, wobei das Cellulosesubstrat eine oder mehrere Cellulosequellen, ausgewählt aus der Gruppe, bestehend aus Holz, Holzpulpe, Papierherstellungsschlamm, Abfallströmen von Papierpulpe, Spanplatte, Maisstroh, Maisfaser, Maiskolben, Reis, Abfall von Papier- und Zellstoffverarbeitung, holzigen oder krautigen Pflanzen, Fruchtpulpe, Gemüsepulpe, Bimsstein, Getreideschlempe, Gräsern, Reishülsen, Zuckerrohrbagasse, Baumwolle, Jute, Hanf, Flachs, Bambus, Sisal, Abaca, Stroh, Maiskolben, Blättern, Weizenstroh, Kokosnusshaar, Algen, Hirse und Gemischen davon, umfasst.

3. Verfahren nach Anspruch 1, wobei das Cellulosesubstrat mechanisch oder chemisch vorbehandelt ist.

4. Verfahren nach Anspruch 1, wobei der fermentierende Mikroorganismus eine Hefe- oder Bakterienzelle ist.

5. Verfahren nach Anspruch 1, wobei der fermentierende Mikroorganismus ein ethanologener Mikroorganismus ist.

6. Verfahren nach Anspruch 1, wobei die organische Substanz Alkohol ist.

7. Verfahren nach Anspruch 1, wobei die organische Substanz Ethanol ist.

8. Reaktionsfähige Zusammensetzung zur Erzeugung einer organischen Substanz, umfassend ein Gemisch von einem Cellulosesubstrat, Gesamtfermentationsbrühe und fermentierendem Mikroorganismus, wobei die reaktionsfähige Zusammensetzung frei von ergänzender Stickstoffquelle ist,
wobei die Gesamtfermentationsbrühe durch Kultivierung eines Fadenpilzes, verwendbar für den Abbau eines Cellulosesubstrats, hergestellt wird.

9. Reaktionsfähige Zusammensetzung nach Anspruch 8, wobei das Cellulosesubstrat eine oder mehrere Cellulosequellen, ausgewählt aus der Gruppe, bestehend aus Holz, Holzpulpe, Papierherstellungsschlamm, Abfallströmen von Papierpulpe, Spanplatte, Maisstroh, Maisfaser, Maiskolben, Reis, Abfall von Papier- und Zellstoffverarbeitung, holzigen oder krautigen Pflanzen, Fruchtpulpe, Gemüsepulpe, Bimsstein, Getreideschlempe, Gräsern, Reishülsen, Zuckerrohrbagasse, Baumwolle, Jute, Hanf, Flachs, Bambus, Sisal, Abaca, Stroh, Maiskolben, Blättern, Weizenstroh, Kokosnusshaar, Algen, Hirse und Gemischen davon, umfasst.

10. Reaktionsfähige Zusammensetzung nach Anspruch 8, wobei das Cellulosesubstrat mechanisch oder chemisch vorbehandelt ist.

11. Reaktionsfähige Zusammensetzung nach Anspruch 8, wobei der fermentierende Mikroorganismus eine Hefe- oder Bakterienzelle ist.

12. Reaktionsfähige Zusammensetzung nach Anspruch 8, wobei der fermentierende Mikroorganismus ein ethanologener Mikroorganismus ist.

## Revendications

1. Procédé de production d'une substance organique par saccharification et fermentation simultanées, comprenant:
(a) la combinaison, en l'absence d'une source d'azote supplémentaire, d'un substrat cellulosique, d'un bouillon de fermentation entier et d'un micro-organisme de fermentation; et
(b) l'incubation du substrat cellulosique, du bouillon de fermentation entier et du micro-organisme de fermentation dans des conditions propices tant à l'hydrolyse de la cellulose en glucose et/ou xylose qu'à la conversion du glucose et/ou xylose en substance organique,
dans lequel le bouillon de fermentation entier est préparé par culture d'un champignon filamenteux utile pour la dégradation d'un substrat cellulosique.

2. Procédé selon la revendication 1, dans lequel le substrat cellulosique comprend une ou plusieurs sources de cellulose choisies dans le groupe constitué par le bois, une pâte de bois, une boue de fabrication de papier, les courants résiduaires de pâte de bois, un panneau de particules, une paille de maïs, une fibre de maïs, un épi de maïs, le riz, un résidu de traitement de papier et de pâte, les plantes ligneuses ou herbacées, une pulpe de fruit, une pulpe végétale, une pierre ponce, les drêches de distillerie, les graminées, les balles de riz, une bagasse de canne à sucre, le coton, le jute, le chanvre, le lin, le bambou, le sisal, l'abaca, la paille, les épis de maïs, les feuilles, la paille de blé, les cheveux de noix de coco, les algues, le panic raide, et des mélanges de ceux-ci.

3. Procédé selon la revendication 1, dans lequel le substrat cellulosique est prétraité mécaniquement ou chimiquement.

4. Procédé selon la revendication 1, dans lequel le micro-organisme de fermentation est une levure ou une cellule bactérienne.

5. Procédé selon la revendication 1, dans lequel le micro-organisme de fermentation est un micro-organisme éthanologène.

6. Procédé selon la revendication 1, dans lequel la substance organique est un alcool.

7. Procédé selon la revendication 1, dans lequel la substance organique est l'éthanol.

8. Composition réactive pour la production d'une substance organique comprenant un mélange d'un substrat cellulosique, d'un bouillon de fermentation entier et d'un micro-organisme de fermentation, laquelle composition réactive est exempte de source d'azote supplémentaire,
dans laquelle le bouillon de fermentation entier est préparé par culture d'un champignon filamenteux utile pour la dégradation d'un substrat cellulosique.

9. Composition réactive selon la revendication 8, dans laquelle le substrat cellulosique comprend une ou plusieurs sources de cellulose choisies dans le groupe constitué par le bois, une pâte de bois, une boue de fabrication de papier, les courants résiduaires de pâte de bois, un panneau de particules, une paille de maïs, une fibre de maïs, un épi de maïs, le riz, un résidu de traitement de papier et de pâte, les plantes ligneuses ou herbacées, une pulpe de fruit, une pulpe végétale, une pierre ponce, les drêches de distillerie, les graminées, les balles de riz, une bagasse de canne à sucre, le coton, le jute, le chanvre, le lin, le bambou, le sisal, l'abaca, la paille, les épis de maïs, les feuilles, la paille de blé, les cheveux de noix de coco, les algues, le panic raide, et des mélanges de ceux-ci.

10. Composition réactive selon la revendication 8, dans laquelle le substrat cellulosique est prétraité mécaniquement ou chimiquement.

11. Composition réactive selon la revendication 8, dans laquelle le micro-organisme de fermentation est une levure ou une cellule bactérienne.

12. Composition réactive selon la revendication 8, dans laquelle le micro-organisme de fermentation est un micro-organisme éthanologène.
